# EUROPEAN PATENT SPECIFICATION

(11) **EP 1 013 252 B1**
(45) Date of publication and mention of the grant of the patent: **24.01.2007**
(21) Application number: 99125809.6
(22) Date of filing: 23.12.1999
(51) Int. Cl.: A61F 13/15

(54) **Absorbent article with superabsorbent particles and with densified region and manufacturing method**
Saugfähiger Gegenstand mit hochabsorbierenden Teilchen und mit einer verdichteten Zone und Herstellverfahren
Article absorbant avec particules de superabsorbants et avec une région comprimée et sa méthode de fabrication

(30) Priority: 23.12.1998 US 219987
(43) Date of publication of application: 28.06.2000
(73) Proprietor: McNEIL-PPC, Inc., Skillman, New Jersey 08558 (US)
(72) Inventor: Rosenfeld, Leonard G., East Windsor, NJ 08520 (US); Worringer, Steven S., East Windsor, NJ 08520 (US)
(74) Representative: Metten, Karl-Heinz

(56) References cited:
- EP-A- 0 769 284
- EP-A- 0 781 537
- US-A- 4 259 387

## Description

### FIELD OF THE INVENTION

The invention relates to an absorbent article, specifically a feminine hygiene article, having emulsion polymerized superabsorbent particles and at least one highly densified region and a method for its manufacture, both known, for instance from US-A-4 259 387. The article comprises an absorbent element which comprises a combination of cellulosic fibers and superabsorbent polymer particles with substantially rounded surfaces, and a continuous film barrier layer. The article further has at least one densified region with a density of at least 0.5 g/cc for stabilization during use.

### BACKGROUND OF THE INVENTION

Absorbent articles such as sanitary napkins or feminine hygiene articles typically have an absorbent element that has a body-facing surface adapted to receive body liquid into the absorbent element, and a liquid-impervious garment-facing surface that acts as a barrier to body liquid to prevent the liquid from staining the user's clothing. Such absorbent elements have traditionally been made from readily available and relatively inexpensive cellulosic absorbent materials such as cotton fibers, wood pulp fluff, cellulosic tissue or wadding, or other absorbent fibers. These materials have provided satisfactory absorbency of liquids both in terms of absorbency rate and overall absorbent capacity. Unfortunately, absorbent elements made from such cellulosic absorbent materials may tend to collapse when wetted, thereby losing some of their void volume. Additionally, this so-called wet-collapse often leads to bunching, roping, and splitting of the absorbent element, further reducing its absorbent capability and its comfort to the user. Such structures may also allow absorbed liquid to be squeezed back out of the structure onto the skin or clothing of the user of the absorbent article. This squeeze-out also contributes to a wet, uncomfortable feeling against the wearer's skin.

Various means have been utilized to help alleviate the problems caused by wet-collapse of cellulosic absorbent structures. Among these are the preshaping of the article to more closely approximate the contours of the user's perineal region, as taught in U.S. 4,654,040, the addition of resilient materials, as described in U.S. 3,060,936, and the formation of a densified layer as described U.S. Patent 4,217,901.

Recently, superabsorbent polymer particles have been combined with the more traditional absorbent materials to provide structures with enhanced absorbency and retention, which may help to eliminate the problems of squeeze-out and wet surface feel. A drawback to superabsorbent polymer particles, however, is their relatively high cost compared to the more traditional absorbent materials. Additionally, since superabsorbent polymer particles tend to swell as they absorb liquid, they must be properly placed within an absorbent structure to allow for this swelling and to most fully utilize their absorbent capacity.

Although the addition of superabsorbent polymer particles to cellulosic absorbent structures may enhance the absorbency of the structures, the superabsorbent addition alone does nothing to improve the stability of the cellulosic structure in comparison to cellulosic structures without superabsorbent polymer particles. Therefore, absorbent products containing cellulosic absorbent structures containing superabsorbent particles must be treated or otherwise configured to help alleviate the problems caused by the wet-collapse of the cellulosic fibers. A desired method of helping to prevent this wet-collapse is the formation of densified stabilizing regions in the absorbent structure. Unfortunately, the formation of densified stabilizing regions in articles made with absorbent elements containing superabsorbent particles may compromise the liquid-impervious garment-facing surface of the article, since the densification process tends to push the superabsorbent particles against the liquid-impervious barrier film surface, thus causing tiny holes or tears, commonly known as pinholes, to form in the film surface. The presence of these holes or tears is undesirable as it allows the passage of liquid and diminishes the liquid-impervious nature of the garment-facing surface of the article.

One solution to this problem of pinhole-formation in the liquid-impervious surface from the superabsorbent particles is to add the liquid-impervious surface as a separate layer after the densification of the superabsorbent containing absorbent element. Unfortunately, the addition of the liquid-impervious layer to the absorbent element after the densification produces poor conformance between the liquid-impervious layer and the absorbent element. This poor conformance may undermine the stabilization that the densification is intended to overcome.

Another solution to the pinhole formation problem involves the use of superabsorbent particles to which have been added a plasticizer such as glycerol or a small amount of water to soften the particles somewhat and to allow the particles to become more pliable without diminishing their absorbent capacity. The softening of the particles allows for the densification process to be carried out with the impermeable barrier in place without creating pinholes in the barrier. The softened particles are able to conform under the densification pressure and therefore, do not push through the impermeable barrier layer in the same way that dry particles might. However, the softened particles may provide handling or processing difficulties since they may tend not to flow in the same way that a relatively dry powder would flow.

Therefore, what is needed is an absorbent article with a continuous liquid-impervious layer and an absorbent element containing superabsorbent polymer particles, and having densified stabilization regions.

### SUMMARY OF THE INVENTION

It is an object of the current invention to provide an absorbent article with an absorbent element containing superabsorbent particles and having densified stabilizing regions, and with a continuous, intact liquid-impervious barrier layer with very few or no tears or holes. This object is accomplished features according to claims 1 and 7, e.g. by the inclusion of superabsorbent polymer particles with substantially rounded surfaces, such as emulsion polymerized superabsorbent polymer particles, in the absorbent element and the subsequent densification of certain areas of the absorbent element to form densified stabilizing regions. The densified stabilizing regions must be densified to such an extent that the article does not lose its stability when it is worn or becomes wet. Such desirable density of the densified stabilizing regions is at least 0.5 g/cc. The superabsorbent polymer particles contain less than 15% by weight of moisture.

### DETAILED DESCRIPTION OF THE DRAWINGS

Figure 1 is a plan view of a preferred embodiment of the absorbent article of the present invention for use by a woman for feminine hygiene.
Figure 2 is a cross-sectional view taken along line II - II of Figure 1.
Figures 3a through 3e are examples of typical round particle and agglomerate morphologies.
Figures 4a through 4e are examples of typical jagged particle morphologies.

### DETAILED DESCRIPTION OF PREFERRED EMBODIMENTS

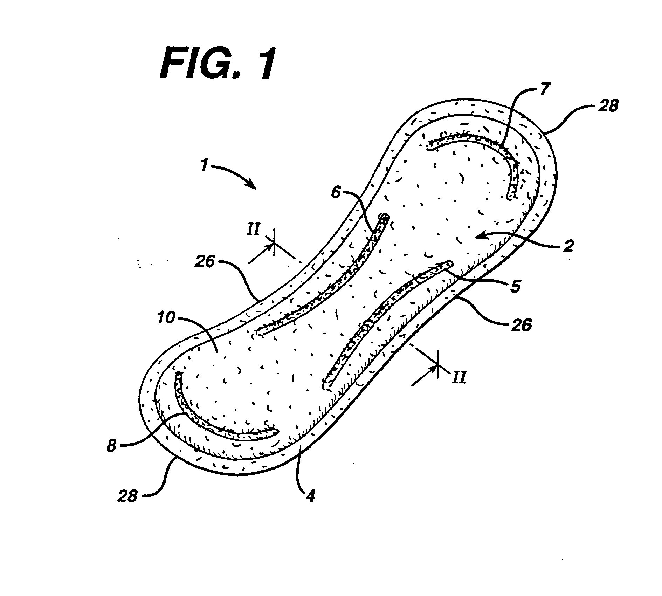

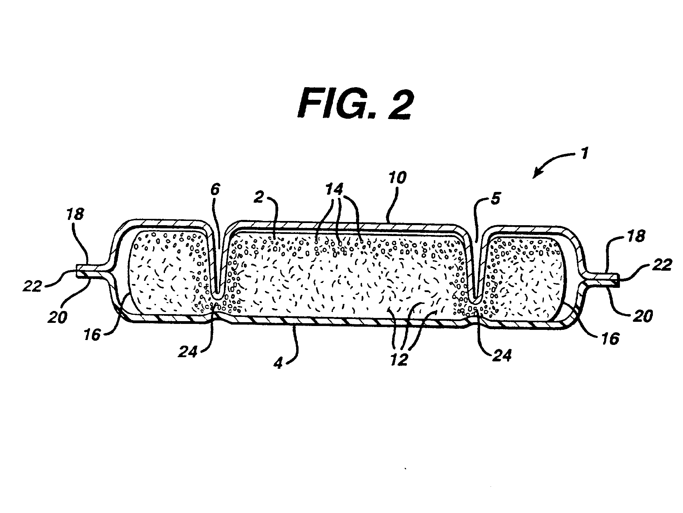

An absorbent article for use by a woman for feminine hygiene according to this invention is shown in Figure 1. The article 1 is comprised of a generally rectangular absorbent structure 2 overlying a continuous barrier film layer 4. The article has densified stabilizing regions that take the form of densified channels 5, 6, 7, 8 along the sides and ends of the article. A liquid permeable body-facing cover layer 10 may cover the top surface of the article. As is shown in Figure 2, the absorbent structure 2 comprises a combination of cellulosic fibers 12 and superabsorbent polymer particles with substantially rounded surfaces 14.

The surface of the article that is intended to be worn against the body of the user may be formed by a layer of a body-fluid pervious material, typically referred to as a cover or facing. The cover may be formed from any liquid pervious material that is comfortable against the skin and that permits fluids to penetrate to the underlying absorbent structure, which then absorbs and retains the fluid. The cover should generally retain little or no fluid within its structure in order to provide a relatively dry surface next to the skin. The fluid pervious cover may be a fibrous nonwoven fabric made from fibers or filaments of polymers such as polyethylene, polypropylene, polyester, or cellulose. Alternatively, the cover may be formed from an apertured polymeric film. The thickness of the cover may vary from 0.001 to 0.062 inch, depending upon the material chosen.

The surface of the article that is intended to be worn away from the body of the wearer and against the wearer's undergarment comprises a layer of body fluid impermeable material, typically referred to a barrier. This impervious barrier comprises any thin, flexible body fluid impermeable material such as a polymeric film-for example, polyethylene, polypropylene, polyester, polyurethane, or cellophane. The thickness of a barrier of such a polymeric film is typically only about 0.001 to 0.002 inch.

The absorbent structure of the article comprises a combination of superabsorbent polymer particles and cellulosic fibers. The cellulosic fibers may be, without limitation, wood pulp, regenerated cellulose fibers, rayon fibers, peat moss fibers, or cotton fibers. The cellulosic fibers are preferably randomly and loosely associated, as in an airlaid structure, or they may be associated together into an oriented structure, such as a carded web. Alternatively, the cellulosic fibers may be wetlaid to form a relatively low density structure capable of having portions thereof densified to a density of at least 0.5 g/cc. Additionally, other absorbent or nonabsorbent materials may be added to the absorbent structure. Examples of such other materials include, without limitation, synthetic resilient fibers, binder fibers, odor controlling fibers or particles, perfumes, synthetic binder compounds such as ethylene vinyl acetate and poly (vinyl alcohol), and the like.

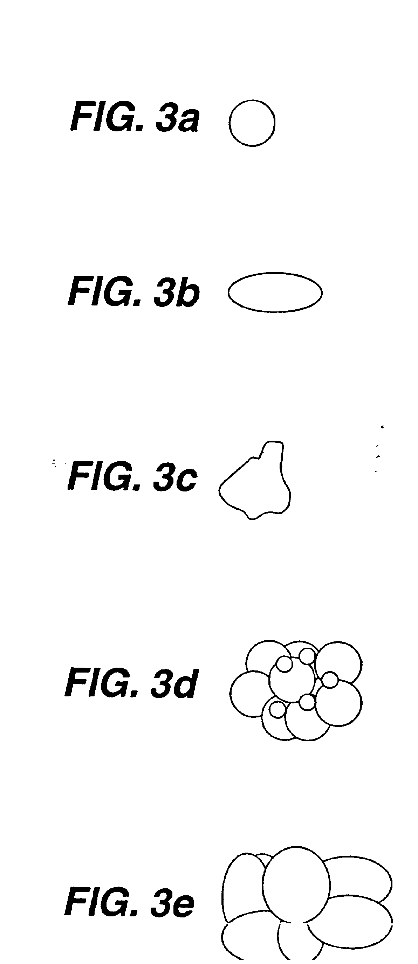

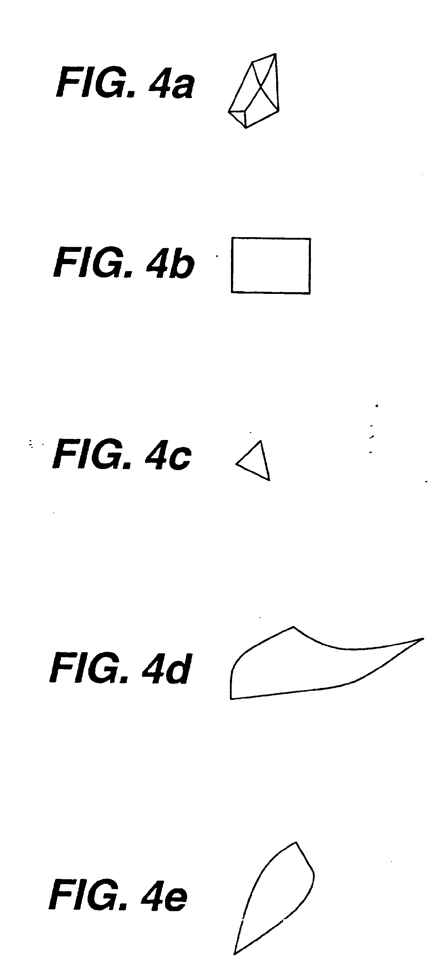

The cellulosic fibers are combined with superabsorbent polymer particles to form the article's absorbent structure. The superabsorbent polymer particles comprise small round particles or agglomerates of tiny rounded particles. In any case, the superabsorbent particles have surfaces that are substantially rounded. By "substantially rounded", it is meant that the surfaces of the particles are generally free of jagged, sharp, or pointed edges. Figures 3a through 3e show examples of typical round particle and agglomerate morphologies. Preferably, these particles with substantially rounded surfaces are emulsion polymerized superabsorbent polymer particles. In any case, the superabsorbent polymer particles used in this invention have generally rounded cross-sectional shapes and surfaces which are substantially free of sharp, jagged, or pointed edges. Examples of suitable emulsion polymerized superabsorbent polymer particles are Sumitomo SA60N™, and Sumitomo J550™, which are available from Sumitomo Seika Chemicals Co., Ltd. Of Osaka, Japan. In contrast, solution polymerized superabsorbent particles have generally jagged, uneven, or sharp edges on their surfaces. Examples of typical jagged particle morphologies are illustrated in Figures 4a through 4e. A more complete discussion of the emulsion polymerization process and the solution polymerization process is disclosed in U.S. Re. 32,649 to Brandt *et al*, which is herein incorporated by reference.

The fibers and particles may be combined in any of several ways. The particles may be substantially homogeneously blended with the fibers throughout the absorbent structure. Alternatively, the particles may be present within a distinct zone within the absorbent structure.

In some cases, it may be desirable to locate the superabsorbent particles within a specific and distinct zone within the absorbent structure. The boundaries of these zones may be located within the x-y plane or in the z-direction of the structure. For instance, the superabsorbent particles may be concentrated in the upper portion of the structure's thickness as illustrated in Figure 2. Concentrating the superabsorbent particles in the upper portion of the structure's thickness in this way further aids in the prevention of pinhole formation. Alternatively, the superabsorbent particles may be located throughout the structure's thickness but concentrated in the central portion of the structure where the fluid is expected to enter the structure, leaving the ends substantially free of superabsorbent. Furthermore, the superabsorbent particles may be concentrated both in the upper portion of the structure's thickness and only within the central portion of the structure, leaving the ends substantially free of superabsorbent particles. Methods of forming such zoned distributions of superabsorbent particles are described in US Patents 5,004,579, 5,213,817, 5,350,817, ad 5,614,147 which are incorporated herein by reference.

As shown in the figures, the absorbent structure 2 is contained between the article's facing or cover layer 10 and the barrier layer 4. The cover layer 10 and barrier layer 4 preferably extend beyond the edges 16 of the absorbent structure to form peripheral cover extensions 18 and barrier extensions 20. These peripheral cover extensions 18 and barrier extensions 20 are joined to one another to form a flange seal 22 around the outside edges of the article. The flange seal may be formed by any sealing means known in the art, including without limitation, heat sealing, ultrasonic sealing, adhesive sealing, or mechanical sealing. Although the preferred article of this invention is made with a flange seal, such a method of sealing the edges of the article is not necessary to the invention. The edges of the article may also be sealed by means of wrapping the cover layer, the barrier layer, or both layers around the edges.

An important feature of the article of the invention is the presence of at least one densified region with a density of at least 0.5 g/cc. As shown in the figures, the at least one densified region preferably takes the form of at least one channel 5, 6, 7, 8 that is embossed or compressed into the article to a density, measured at the bottom 24, most dense portion of the channel, of at least about 0.5 g/cc. Preferably, the density is between about 0.5 g/cc and 1.3 g/cc. Densified regions with densities less than 0.5 g/cc do not maintain the necessary stability in use, as the material in the densified portion of the channel may "spring back" or "recover" when the article is subjected to normal wear conditions and moisture provided by perspiration and body fluid discharges. However, when the densified region is created with a density of at least 0.5 g/cc, it retains its density in use, greatly enhances the stability of the article in use, and helps to prevent twisting and bunching when the article becomes wet and is subjected to the pressures of ordinary wear.

The densified region is compressed or embossed into the article with at least the barrier layer in place against a surface of the absorbent structure. The presence of the barrier layer against the absorbent structure during the densification process has several advantages. The presence of the barrier during the densification process helps to maintain the integrity of the absorbent structure during the densification; it allows for the formation of uniformly densified regions; and it enhances the overall stability of the absorbent article. It is also preferable that the cover layer be positioned against the body-facing surface of the absorbent structure when the at least one densified region is created; however, this is not necessary.

The densification process essentially compresses regions of the absorbent structure, which has an uncompressed density of between 0.01 and 0.05 g/cc, to a density of at least 0.5 g/cc, and preferably to a density of at least 0.9 g/cc. The densified regions may be in any pattern. In the preferred embodiment of the invention illustrated in Figure 1, the densified regions are present as two longitudinal arcs 5, 6 and two lateral arcs 7, 8. The longitudinal arcs 5, 6 extend generally along the longitudinal sides 26 of the article and are oriented so that they curve generally inwardly toward each other. The lateral arcs 7, 8 extend generally laterally across the ends 28 of the article and are oriented so that they curve generally outwardly away from each other. Each arc-shaped densified region is typically less than about 0.25 inches wide at its bottom, most dense portion. The longitudinally extending arcs are generally between about 25 and about 100 millimeters in length, and the laterally extending arcs are generally between about 25 and about 75 millimeters in length.

The densified regions may be formed in the article by any known means of densification, including embossing or calendering. Performing the densification at an elevated temperature aids in the formation of the densified region. A preferred method of forming the densified regions of the present invention comprises passing the absorbent article between a heated embossing roll and an anvil roll. The embossing roll has raised portions corresponding to the desired pattern of the densified regions. The anvil roll has a smooth surface. The two rolls are separated by a predetermined space so that a gap is created between the anvil roll and the raised portions of the embossing roll. The size of the gap depends upon the thickness of the undensified absorbent article and is set such that the passage of an absorbent article between the rolls results in the formation of densified regions with a density of at least 0.5 g/cc.

According to an important aspect of the present invention, superabsorbent particles with substantially rounded surfaces are used in the absorbent structure to minimize the formation of pinholes in the film barrier layer under the densified regions. Experimental tests were done with sample absorbent structures made with emulsion polymerized superabsorbent particles, with solution polymerized superabsorbent particles, and with no superabsorbent particles (control) to determine how many pinholes were formed in the barrier film layers. Each sample was made of a blend of 0.5 g superabsorbent and 5.5 g of pulp formed into a generally rectangular absorbent structure and contained between a layer of 1 mil thick polyethylene film and a nonwoven cover layer comprising polypropylene staple fibers. The film is commercially available from Edison Plastics Company of Newport News, VA, as XP 1123. The nonwoven cover layer is commercially available from Stearns of Cincinnati, Ohio, as T1046. Fifty samples of each type were passed between an anvil roll and an embossing roll to form densified areas in a pattern similar to that shown in Figure 1. The gap between the anvil roll and the raised areas on the embossing roll was set at 9/14 mil. The total number of pinhole stains for all 50 samples of each type was determined according to the Pinhole Stain Test as described below.

The Pinhole Stain Test is conducted by attaching the positioning adhesive of an absorbent article to a layer of washed, Alpha Mills white cotton fabric of a sufficient size to completely cover the densified areas and the positioning adhesive. A quantity of 2.5 cc of red dye solution is then applied to each densified region via the body-facing or cover surface of each article. The red dye solution comprises 50 grams of Calico Scarlet 2 RB red powder available from Pylam Inc. of Tempe Arizona, mixed with 5.0 gallons of water. A pressure roll apparatus consisting of two weighted rollers mounted on an axle with a total weight of 10 pounds is rolled once over the product such that one roller covers each longitudinal densified region. The apparatus is then rolled once again over the product such that one roller covers each lateral densified region. The fabric is then examined for any stains caused by transfer of the red dye solution through the barrier layer. Stains in the fabric indicate the presence of pinholes.

Table I shows the results of the experiments.

**TABLE I**

| **Superabsorbent** | **Particle Size** | **Polymerization Method** | **Particle Surface** | **Number of Pinhole Stains** |
|---|---|---|---|---|
| Chemdal L2100a | 0.25 mm | Solution | Jagged | 48 |
| Sumitomo SA60N | 0.26 mm | Emulsion | Round | 10 |
| J550 | 0.15 mm | Emulsion | Round | 0 |
| No Superabsorbent | ----- | ----- | ----- | 0 |

As Table I shows, the articles made with superabsorbent particles with substantially rounded surfaces of a given size had fewer pinhole stains than the articles made with solution polymerized superabsorbent particles of the same size. Furthermore, the articles made with smaller particles of superabsorbent particles with substantially rounded surfaces had no pinhole stains, which was comparable to the number of pinhole stains in articles made without superabsorbent.

The specification and embodiments above are presented to aid in the complete and non-limiting understanding of the invention disclosed herein. The invention resides in the claims hereinafter appended.

## Claims

1. An absorbent article for use by a woman for feminine hygiene, the article comprising:
- a cellulosic fibers (12);
- a continuous film barrier layer (4);
- at least one densified region (5, 6, 7, 8) having a density of at least 0.5 g/cc, **characterized in that** the article further comprises superabsorbent polymer particles with substantially rounded surfaces (14).

2. The absorbent article of claim 1 wherein the superabsorbent polymer particles comprise emulsion polymerized superabsorbent polymer particles.

3. The absorbent article of claim 1 wherein the at least one densified region (5, 6, 7, 8) has a densitiy of at least 0.9 g/cc.

4. The absorbent article of claim 1 wherein the at least one densified region (5, 6, 7, 8) comprises superabsorbent polymer particles with substantially rounded surfaces (14).

5. The absorbent article of claim 1 wherein the at least one densified region comprises two compressed side channels (5, 6).

6. The absorbent article of claim 1 further comprising a first surface and a second surface separated by a thickness, wherein the superabsorbent polymer particles with substantially rounded surfaces (14) are located within a portion of the thickness that is closer to the first surface than to the second surface.

7. A method of making an absorbent article for placement in a human perineal region for absorption of body fluid, the method comprising:
- providing an absorbent structure (2) comprising a combination of cellulosic fibers (12) and superabsorbent polymer particles with substantially rounded surfaces (14), the structure (2) having a first surface, an opposite second surface, and a density of less than 0.5 g/cc;
- providing a barrier layer (4) comprising a layer of a liquid impermeable film;
- placing the barrier layer (4) against the second surface of the absorbent structure (2); and
- densifying a portion of the first surface of the absorbent structure (2) to form a densified region with a density of at least about 0.5 g/cc.

8. The method according to claim 7 wherein the superabsorbent polymer particles comprise emulsion polymerized superabsorbent polymer particles.

9. The method according to claim 7 wherein the first surface is densified to form a densified region with a density of at least about 0.9 g/cc.

10. The method according to claim 7 wherein the combination of cellulosic fibers (12) and superabsorbent polymer particles (14) is a uniform blend of cellulosic fibers and superabsorbent polymer.

## Patentansprüche

1. Saugfähiger Gegenstand für die Benutzung durch eine Frau für Frauenhygiene, wobei der Gegenstand umfaßt:
- Zellulosefasern (12);
- eine durchgängige Folienbarrierelage (4);
- zumindest eine verdichtete Zone (5, 6, 7, 8) mit einer Dichte von zumindest 0,5 g/cc, **dadurch gekennzeichnet, daß** der Gegenstand ferner superabsorbierende Polymerteilchen mit im wesentlichen gerundeten Oberflächen (14) umfaßt.

2. Saugfähiger Gegenstand nach Anspruch 1, **dadurch gekennzeichnet, daß** die superabsorbierenden Polymerteilchen emulsionspolymerisierte superabsorbierende Polymerteilchen umfassen.

3. Saugfähiger Gegenstand nach Anspruch 1, **dadurch gekennzeichnet, daß** die zumindest eine verdichtete Zone (5, 6, 7, 8) eine Dichte von zumindest 0,9 g/cc aufweist.

4. Saugfähiger Gegenstand nach Anspruch 1, **dadurch gekennzeichnet, daß** die zumindest eine verdichtete Zone (5, 6, 7, 8) superabsorbierende Polymerteilchen mit im wesentlichen gerundeten Oberflächen (14) umfaßt.

5. Saugfähiger Gegenstand nach Anspruch 1, **dadurch gekennzeichnet, daß** die zumindest eine verdichtete Zone zwei komprimierte Seitenkanäle (5, 6) umfaßt.

6. Saugfähiger Gegenstand nach Anspruch 1, der weiterhin eine durch eine Dicke getrennte erste Oberfläche und eine zweite Oberfläche aufweist, **dadurch gekennzeichnet, daß** die superabsorbierenden Polymerteilchen mit im wesentlichen gerundeten Oberflächen (14) sich innerhalb eines Abschnitts der Dicke befinden, der dichter an der ersten Oberfläche als an der zweiten Oberfläche liegt.

7. Verfahren für die Herstellung eines saugfähigen Gegenstandes für das Einlegen in den Schrittbereich eines Menschen zur Absorption von Körperflüssigkeit, wobei das Verfahren umfaßt:
- Bereitstellung einer saugfähigen Struktur (2), die eine Kombination von Zellulosefasern (12) und superabsorbierenden Polymerteilchen mit im wesentlichen gerundeten Oberflächen (14) umfaßt, wobei die Struktur (2) eine erste Oberfläche, eine gegenüberliegende zweite Oberfläche, und eine Dichte von weniger als 0,5 g/cc aufweist;
- Bereitstellung einer Barrierelage (4), die eine Lage einer flüssigkeitsundurchlässigen Schicht umfaßt;
- Platzieren der Barrierelage (4) gegen die zweite Oberfläche der saugfähigen Struktur (2); und
- Verdichten eines Abschnitts der ersten Oberfläche der saugfähigen Struktur (2) zur Ausbildung einer verdichteten Zone mit einer Dichte von zumindest zirka 0,5 g/cc.

8. Verfahren nach Anspruch 7, **dadurch gekennzeichnet, daß** die superabsorbierenden Polymerteilchen emulsionspolymerisierte superabsorbierende Polymerteilchen umfassen.

9. Verfahren nach Anspruch 7, **dadurch gekennzeichnet, daß** die erste Oberfläche verdichtet ist, um eine verdichtete Zone mit einer Dichte von zumindest zirka 0,9 g/cc zu bilden.

10. Verfahren nach Anspruch 7, **dadurch gekennzeichnet, daß** die Kombination von Zellulosefasern (12) und von superabsorbierenden Polymerteilchen (14) eine einheitliche Mischung von Zellulosefasern und superabsorbierendem Polymer ist.

## Revendications

1. Article absorbant destiné à l'hygiène féminine, l'article comprenant :
- des fibres de cellulose (12);
- une couche de film continue servant de barrière (4) ;
- au moins une région densifiée (5, 6, 7, 8) ayant une masse volumique d'au moins 0,5 g/cm³, **caractérisée en ce que** l'article comprend en outre des particules de polymère superabsorbantes avec des surfaces essentiellement arrondies (14).

2. Article absorbant selon la revendication 1 dans lequel les particules de polymère superabsorbantes comprennent des particules superabsorbantes de polymère polymérisé en émulsion.

3. Article absorbant selon la revendication 1 dans lequel au moins une des régions densifiées (5, 6, 7, 8) a une masse volumique d'au moins 0,9 g/cm³.

4. Article absorbant selon la revendication 1 dans lequel au moins une des régions densifiées (5, 6, 7, 8) comprend des particules de polymère superabsorbantes avec des surfaces essentiellement arrondies (14).

5. Article absorbant selon la revendication 1 dans lequel au moins une des régions densifiées comprend deux canaux latéraux compressés (5, 6).

6. Article absorbant selon la revendication 1 comprenant en outre une première et une deuxième surfaces séparées par une épaisseur, dans laquelle les particules de polymère superabsorbantes avec des surfaces essentiellement arrondies (14) se trouvent à l'intérieur d'une partie de l'épaisseur qui est plus près de la première surface que de la deuxième.

7. Procédé de fabrication d'un article absorbant destiné à être placé en contact avec une région périnéale de l'humain pour l'absorption de fluide corporel, le procédé comprenant les étapes consistant à :
- fournir une structure absorbante (2) comprenant une combinaison de fibres de cellulose (12) et des particules de polymère superabsorbantes avec des surfaces essentiellement arrondies (14), la structure (2) ayant une première surface, une deuxième surface opposée, et une masse volumique inférieure à 0,5 g/cm³ ;
- fournir une couche barrière (4) comprenant une couche de film imperméable aux liquides ;
- placer la couche barrière (4) contre la deuxième surface de la structure absorbante (2) ; et
- densifier une partie de la première surface de la structure absorbante (2) afin de former une région densifiée avec une masse volumique d'au moins environ 0,5 g/cm³.

8. Procédé selon la revendication 7 dans lequel les particules de polymère superabsorbantes comprennent des particules superabsorbantes de polymère polymérisé en émulsion.

9. Procédé selon la revendication 7 dans lequel la première surface est densifiée afin de former une région densifiée d'une masse volumique d'au moins environ 0,9 g/cm³.

10. Procédé selon la revendication 7 dans lequel la combinaison des fibres de cellulose (12) et les particules de polymère superabsorbantes (14) sont un mélange uniforme de fibres de cellulose et de polymère superabsorbant.
